# EUROPÄISCHE PATENTSCHRIFT

(11) **EP 3 795 080 B1**
(45) Veröffentlichungstag und Bekanntmachung des Hinweises auf die Patenterteilung: **11.01.2023**
(21) Anmeldenummer: 19198383.2
(22) Anmeldetag: 19.09.2019
(51) Int. Cl.: A61B 6/00, G01N 23/087, A61K 49/04, A61M 5/00

(54) **KONTRASTMITTELBASIERTE ZEITKODIERUNG IN DER RÖNTGENBILDGEBUNG**
CONTRAST MEDIA BASED TIME CODING IN X-RAY IMAGING
CODAGE TEMPOREL À BASE D'AGENT DE CONTRASTE DANS L'IMAGERIE PAR RAYONS X

(43) Veröffentlichungstag der Anmeldung: 24.03.2021
(73) Patentinhaber: Siemens Healthcare GmbH, 91052 Erlangen (DE)
(72) Erfinder: Flohr, Thomas, 91486 Uehlfeld (DE); Schmidt, Bernhard, 90766 Fürth (DE)

(56) Entgegenhaltungen:
- US-A1- 2010 135 557
- US-A1- 2011 097 273
- US-A1- 2016 113 609
- US-A1- 2018 303 436
- US-A1- 2019 239 842

## Beschreibung

Die Erfindung betrifft ein Bildgebungsverfahren. Bei dem Bildgebungsverfahren erfolgt eine Darstellung funktioneller Daten von einem Untersuchungsbereich eines Untersuchungsobjekts mit Hilfe der Röntgenbildgebung. Weiterhin betrifft die Erfindung ein Bilddatengewinnungssystem.

Mit Hilfe moderner bildgebender Verfahren werden häufig zwei-oder dreidimensionale Bilddaten erzeugt, die zur Visualisierung eines abgebildeten Untersuchungsobjekts und darüber hinaus auch für weitere Anwendungen genutzt werden können.

Häufig basieren die bildgebenden Verfahren auf der Erfassung von Röntgenstrahlung, wobei sogenannte Projektionsmessdaten erzeugt werden. Beispielsweise können Projektionsmessdaten mit Hilfe eines Computertomographie-Systems (CT-Systems) akquiriert werden.

Mit Hilfe der CT-Bildgebung konnten lange Zeit "nur" anatomische Strukturen bildlich wiedergegeben werden. Dagegen war die funktionale Bildgebung mittels Computertomographie lange Zeit nicht möglich, unter anderem auch wegen einer zu hohen Strahlenbelastung für den Patienten. In den letzten Jahren aber haben sich dank technologischer Fortschritte die Möglichkeiten zur funktionalen Bildgebung verbessert und ihren Weg in die klinische Routine gefunden. Allerdings sind für die Messung dynamischer Messgrößen, wie zum Beispiel die Blutflussgeschwindigkeit meist eine Vielzahl von Bildern nötig, deren Aufnahme die Strahlenbelastung des Patienten erhöht.

Das Dokument US2018303436 A1 offenbart ein CT-Verfahren, bei welchem zwei unterschiedliche Kontrastmittel in ein Netzwerk von Blutgefäßen injiziert werden.

Das Dokument US2019239842 A1 offenbart ein Verfahren zur Bereitstellung von Bildern zum Nachweis einer Endoleckage, nachdem ein Stentgraft in der Aorta platziert worden ist unter Verwendung von zwei unterschiedlichen Kontrastmitteln

Das Dokument US2010135557 A1 offenbart ein Verfahren zur Auswahl von zwei unterschiedlichen Kontrastmitteln für ein Daul Energy CT Bildgebungsverfahren.

Das Dokument US2016113609 A1 offenbart ein CT-Gerät mit einer Steuerung, die so konfiguriert ist, dass sie eine Differenz zwischen den Abbildungszeitphasen eines ersten Kontrastmittels und eines zweiten Kontrastmittels berechnet.

Das Dokument US2011097273 A1 offenbart ein Verfahren, welches die gleichzeitige Modulation der Verabreichung von mindestens zwei verschiedenen Kontrastmitteln während eines Bildgebungsverfahrens auf der Grundlage eines Modulationsprofils umfasst.

Bei einer Röntgenbildaufnahme werden häufig Kontrastmittel eingesetzt, die dem Patienten injiziert werden, um den Kontrast der Bildaufnahme zu erhöhen und damit eine Diagnose zu erleichtern. Werden spektrale Bildgebungsverfahren, wie zum Beispiel Dual-Energy-Bildgebungsverfahren, genutzt, so kann eine quantitative Verteilung von Kontrastmittel, wie zum Beispiel Iod, in unterschiedlichen anatomischen Bereichen ermittelt werden und auf diese Weise ist grundsätzlich die Gewinnung von funktionellen Informationen möglich.

Allerdings wird damit nur eine Art Schnappschuss einer Kontrastmittelverteilung in einem Patienten zu einem einzigen Zeitpunkt ermittelt. Eine Information über den zeitlichen Verlauf von Kontrastdynamiken ist auf diese Weise nicht verfügbar.

Werden zeitabhängige Prozesse im menschlichen Körper aufgenommen, werden herkömmlich über einen gewissen Zeitraum hinweg aufeinanderfolgende Bildaufnahmen durchgeführt. Beispielsweise wird für die Untersuchung von Gefäßstrukturen eine sogenannte vierdimensionale CTA (CTA = CT-Angiographie) durchgeführt und für die Untersuchung von Parenchym eine Perfusions-CT durchgeführt.

Allerdings gehen mit derartigen Bildgebungsverfahren eine erhöhte Strahlungsdosis und eine lange Aufnahmezeit einher.

Es besteht also das Problem, eine medizintechnische Bildgebung mit möglichst niedriger Strahlenbelastung und kurzer Aufnahmezeit zu entwickeln, mit der dynamische Prozesse dargestellt werden können.

Diese Aufgabe wird durch ein Bildgebungsverfahren gemäß Patentanspruch 1 und ein Bilddatengewinnungssystem gemäß Patentanspruch 10 gelöst.

Bei dem erfindungsgemäßen Bildgebungsverfahren wird eine Konzentration-Zeit-Kurve für eine Injektion von mindestens zwei spektral unterscheidbaren Kontrastmitteln mit einer vorbestimmten zeitlich variablen Konzentration festgelegt. Die Konzentration-Zeit-Kurve gibt Auskunft über die Konzentration der einzelnen injizierten Kontrastmittel in Abhängigkeit von der Zeit. Es werden Röntgenrohdaten, beispielsweise mit Hilfe eines computertomographischen Verfahrens, von einem von den Kontrastmitteln durchfluteten Bereich eines Untersuchungsobjekts aufgenommen. Weiterhin wird eine Spektralzerlegung auf Basis der Röntgenrohdaten bezüglich der unterschiedlichen Kontrastmittel durchgeführt. Mit Hilfe der Spektralzerlegung werden zwei hinsichtlich ihres Röntgenspektrums unterschiedliche, auf den Röntgenrohdaten basierende Datensätze erzeugt, denen jeweils unterschiedliche Kontrastmittel zuzuordnen sind. Anders ausgedrückt, weisen die unterschiedlichen Kontrastmittel jeweils unterschiedliche spektrale Absorptionseigenschaften auf, die bei der Anwendung von Röntgenstrahlung mit unterschiedlichen Röntgenspektren zur Geltung kommen. Werden nun die erfassten Messdaten bzw. Rohdaten bezüglich dieser unterschiedlichen Spektralanteile zerlegt, so können daraus mindestens zwei Bilddatensätze, welche unterschiedlichen Kontrastmitteln zugeordnet sind, auf Basis der Spektralzerlegung rekonstruiert werden. Die beiden Bilddatensätze bilden jeweils von unterschiedlichen Kontrastmitteln besser sichtbar gemachte Strukturen ab. Weiterhin lassen sich anhand der Abschwächungswerte in den Bilddaten die Konzentrationen der unterschiedlichen Kontrastmittel in unterschiedlichen Körperbereichen ermitteln. Weisen nun unterschiedliche anatomische Bereiche dasselbe Mischungsverhältnis bzw. Verhältnis der Konzentrationen der unterschiedlichen Kontrastmittel auf, so ergeben sich für diese Bereiche dieselben Ankunftszeiten der Kontrastmittel. Unterschiedliche Konzentrationsverhältnisse dagegen lassen auf unterschiedliche Ankunftszeiten der Kontrastmittel in unterschiedlichen Bereichen schließen. Diese Zeitkodierung auf Basis der mindestens zwei Bilddatensätze ermöglicht es, Laufzeitdifferenzen von Blutflüssen und eine zeitliche Charakteristik einer Blutverteilung im Körper eines Patienten zu ermitteln und daraus funktionelle Daten zu gewinnen. Die funktionellen Daten bzw. funktionellen Bilddaten repräsentieren im Gegensatz zu anatomischen Bilddaten die physiologische Aktivität von Organen oder Gewebestrukturen. Beispielsweise umfassen die funktionellen Daten Informationen bezüglich der Stoffwechselaktivität oder des Blutflusses eines Untersuchungsobjekts bzw. eines Patienten. Vorteilhaft benötigt man zur Aufnahme der für die Gewinnung der funktionellen Daten notwendigen Bilddaten nur eine zu einem bestimmten Zeitpunkt erzeugte Bildaufnahme anstatt einer Mehrzahl über einen längeren Zeitraum erstellter Bildaufnahmen. Auf diese Weise wird die Strahlenbelastung für den Patienten bei der Bildaufnahme zur Gewinnung physiologischer Daten im Vergleich zu herkömmlich Herangehensweisen reduziert.

Das erfindungsgemäße Bilddatengewinnungssystem weist eine Injektionsplanungseinheit zum Festlegen einer Konzentration-Zeit-Kurve für eine Injektion von mindestens zwei spektral unterscheidbaren Kontrastmitteln mit einer vorbestimmten zeitlich variablen Konzentration auf. Teil des erfindungsgemäßen Bilddatengewinnungssystems ist auch ein Röntgensystem zum Aufnehmen von Röntgenrohdaten von einem von den Kontrastmitteln durchfluteten Bereich eines Untersuchungsobjekts. Das erfindungsgemäße Bilddatengewinnungssystem umfasst auch eine Auswertungseinheit zum Durchführen einer Spektralzerlegung auf Basis der Röntgenrohdaten bezüglich der unterschiedlichen Kontrastmittel. Zudem weist das erfindungsgemäße Bilddatengewinnungssystem eine Rekonstruktionseinheit zum Rekonstruieren von mindestens zwei Bilddatensätzen auf Basis der Spektralzerlegung auf. Überdies umfasst das erfindungsgemäße Bilddatengewinnungssystem eine Datenermittlungseinheit zum Ermitteln von funktionellen Daten auf Basis der mindestens zwei Bilddatensätze. Das erfindungsgemäße Bilddatengewinnungssystem teilt die Vorteile des erfindungsgemäßen Bildgebungsverfahrens.

Die wesentlichen Komponenten des erfindungsgemäßen Bilddatengewinnungssystems können zum überwiegenden Teil in Form von Softwarekomponenten ausgebildet sein. Dies betrifft insbesondere die Injektionsplanungseinheit, die Auswertungseinheit, die Rekonstruktionseinheit und die Datenermittlungseinheit des erfindungsgemäßen Bilddatengewinnungssystems. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete medizintechnische Bildgebungssysteme bzw. Bilddatengewinnungssysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Bilddatengewinnungssystems ladbar ist, mit Programmabschnitten, um die durch Software realisierbaren Schritte des erfindungsgemäßen Bildgebungsverfahrens auszuführen, wenn das Programm in dem Bilddatengewinnungssystem ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zu dem Teilsystem und/oder zur Speicherung an oder in diesem Teilsystem kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen. Die Rechnereinheit kann zum Beispiel Teil eines Terminals oder einer Steuereinrichtung eines bildgebenden Systems, wie zum Beispiel eine CT-Anlage, sein, sie kann aber auch Teil eines entfernt positionierten Serversystems innerhalb eines Datenübertragungsnetzes sein, das mit dem bildgebenden System kommuniziert.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

In einer Variante des erfindungsgemäßen Bildgebungsverfahrens erfolgt nach der Festlegung der Konzentration-Zeit-Kurve der mindestens zwei unterschiedlichen Kontrastmittel eine Injektion der mindestens zwei Kontrastmittel in den Patientenkörper, wobei die Konzentrationen der einzelnen Kontrastmittel in der injizierten Kontrastmittelmischung dem festgelegten Schema der Konzentration-Zeit-Kurve folgen. Vorteilhaft erfolgt bei der Kontrastmittelgabe eine Zeitkodierung, welche es ermöglicht, dynamische Messgrößen hinsichtlich des Blutflussverhaltens im Körper des Patienten mit einer einzigen zu einem einzigen Zeitpunkt erstellten Bildaufnahme zu ermitteln. Auf dies Weise wird die Strahlenbelastung des Patienten im Vergleich zu einer herkömmlichen Vorgehensweise zur Ermittlung dynamischer Messgrößen, bei der eine Mehrzahl sequenzieller Bildaufnahmen erfolgt, reduziert.

Erfindungsgemäß wird durch die Konzentrations-Zeit-Kurve ein Kontrastmittelgemisch der beiden spektral unterscheidbaren Kontrastmittel mit einer vorbestimmten zeitlich variablen Mischkonzentration festgelegt. Diese Gestalt der Konzentrations-Zeit-Kurve eignet sich insbesondere für die Ermittlung dynamischer Größen, wie zum Beispiel die Blutflussrichtung oder die Blutflussgeschwindigkeit. Zudem lässt sich bei dieser Wahl der Konzentrationen der beteiligten Kontrastmittel eine weitgehend homogene Gesamtkonzentration des Kontrastmittels bei der Bilddarstellung erreichen, falls die Kontrastmittelkonzentrationen komplementär gewählt werden, so dass die Gesamtkonzentration der verwendeten Kontrastmittel immer gleich bleibt. Es können auch mehr als nur zwei Kontrastmittel eingesetzt werden und entsprechende funktionelle Daten auf Basis einer Multi-CT-Bildgebung ermittelt werden. Ein möglicher zeitlicher Verlauf der Konzentrationen zweier Kontrastmittel kann zum Beispiel derart gestaltet sein, dass die Konzentration eines ersten Kontrastmittels bei einem Start einer Kontrastmittelgabe bei einem Maximalwert beginnt und im folgenden zeitlichen Verlauf vorzugsweise linear, bis zu einem Minimalwert, beispielsweise bis zu einem Wert von "0", abnimmt. Die Konzentration des zweiten Kontrastmittels dagegen startet mit einem Minimalwert, der vorzugsweise dem Minimalwert der Konzentration des ersten Kontrastmittels entspricht, beispielsweise dem Wert "0", und erhöht sich vorzugsweise linear bis zu einem Maximalwert, der vorzugsweise dem Maximalwert der Konzentration des ersten Kontrastmittels entspricht.

In einer besonders effektiven Ausgestaltung des erfindungsgemäßen Bildgebungsverfahrens umfassen die funktionellen Daten eine dynamische Messgröße. Die dynamische Messgröße kann zum Beispiel eine Blutflussgeschwindigkeit umfassen. Vorteilhaft lässt sich bei Anwendung einer zeitlich variabel gestalteten Konzentrations-Zeit-Kurve mit einer zeitlich variablen Mischkonzentration anhand der jeweils im Bild vorhandenen Kontrastmittelkonzentration auf die Zeit schließen, die das jeweilige Kontrastmittel bis zu diesem Bereich benötigte. Anhand des aus der Bildaufnahme ermittelbaren zurückgelegten Wegs und der ermittelten Zeit lässt sich auf die Blutflussgeschwindigkeit schließen.

Die funktionellen Daten können auch eine Flussrichtung wiedergeben. Auch die Flussrichtung lässt sich bei der Wahl eines Kontrastmittelgemisches mit einer zeitlich variablen Kontrastmittelkonzentration mindestens zweier Kontrastmittel anhand der unterschiedlichen Kontrastmittelkonzentrationen der beiden Kontrastmittel an unterschiedlichen Positionen in Gefäßen ermitteln. Beispielsweise ist die Flussrichtung in Richtung von einer niedrigen zu einer höheren Konzentration des ersten Kontrastmittels zu bestimmen.

In einer Ausgestaltung des erfindungsgemäßen Bildgebungsverfahrens wird die Konzentrations-Zeit-Kurve derart festgelegt, dass die beiden Kontrastmittel zeitlich versetzt zueinander abgegeben werden. Eine derartige Konzentrations-Zeit-Kurve kann zum Beispiel so aussehen, dass zunächst ein erstes Kontrastmittel mit einer zeitlich variablen oder vorzugsweise konstanten Kontrastmittelkonzentration gegeben wird, die Injektion des ersten Kontrastmittels beendet wird und anschließend ein zweites Kontrastmittel ebenfalls mit einer zeitlich variablen oder konstanten Kontrastmittelkonzentration gegeben wird. Eine derartige Vorgehensweise ist zum Beispiel vorteilhaft bei der Ermittlung des Durchblutungsgrades von Gewebebereichen. Dabei können Bereiche mit unterschiedlichen Graden der Durchblutung unterschiedlichen Kontrastmitteln zugeordnet werden. Anders ausgedrückt können mit dieser Gestalt der Konzentration-Zeit-Kurve Bereiche im Gewebe eines Untersuchungsobjekts lokalisiert werden, in denen ein zuerst gegebenes Kontrastmittel verspätet oder gar nicht mehr ankommt.

Die Lokalisierung dieser Bereiche und der damit verbundenen funktionellen Daten werden mithin aus einer räumlichen Variation der Konzentration der Kontrastmittel abgeleitet.

Diese Variante der Konzentrations-Zeit-Kurve kann zum Beispiel zur Ermittlung einer räumlichen Verteilung von Arterien und Venen genutzt werden. Die Bildaufnahme erfolgt dann vorteilhaft zu einem Zeitpunkt, bei dem sich in den Venen das erste Kontrastmittel befindet und in den Arterien sich das später injizierte zweite Kontrastmittel befindet. Auf diese Weise lassen sich Arterien und Venen durch spektral getrennte Bilddatensätze getrennt abbilden oder auch durch entsprechende Farbkodierung übereinandergelegt bzw. in einem gemeinsamen Bild, aber unterschiedlich eingefärbt, darstellen. Insbesondere können die funktionellen Daten Schädelperfusionsmessdaten umfassen. Bei der Ermittlung von Schädelperfusionsmessdaten erfolgt eine zeitlich versetzte Gabe unterschiedlicher Kontrastmittel. Auf der Bilddarstellung finden sich dann beispielsweise Bildbereiche, in denen ein erstes Kontrastmittel vorhanden ist, und Bildbereiche in denen ein zweites Kontrastmittel auftritt, und möglicherweise auch Bildbereiche, in denen überhaupt kein Kontrastmittel auftritt. Die ersten Bereiche können als regulär durchblutete Bereiche des Gehirns interpretiert werden. Die zweiten Bereiche sind schlechter durchblutet, können aber eventuell durch medizinische Maßnahmen noch reaktiviert werden. Die dritten Bereiche sind überhaupt nicht durchblutet und sind daher als unrettbar verloren anzusehen. Vorteilhaft kann mit wenig Aufwand und geringer Strahlendosis eine Schädelperfusion realisiert werden.

Die dynamischen Messgrößen können weiterhin einen Bypassfluss oder einen Rückfluss durch ein Leck in einem Gefäßsystem oder Organ umfassen. Ein solcher Bypassfluss kann zum Beispiel zwischen einem Stent und einer Aussackung, einem sogenannten Aneurysma, auftreten. Die Lokalisierung unterschiedlicher Kontrastmittel kann hier dazu genutzt werden, um zu ermitteln, wo sich das Aneurysma befindet und an welcher Stelle ein Leck auftritt.

Die Erfindung wird im Folgenden unter Hinweis auf die beigefügten Figuren anhand von Ausführungsbeispielen noch einmal näher erläutert. Es zeigen:
FIG 1 ein Flussdiagramm, welches ein Bildgebungsverfahren gemäß einem ersten Ausführungsbeispiel der Erfindung veranschaulicht,
FIG 2 ein Schaubild, welches eine Konzentrationszeitkurve eines Gemischs zweier Kontrastmittel darstellt,
FIG 3 ein Schaubild, welches eine Konzentrationszeitkurve einer zeitversetzten Kontrastmittelgabe darstellt,
FIG 4 eine schematische Darstellung eines Beins mit Arterien und Venen,
FIG 5 eine schematische Darstellung eines Blutgefäßes, dessen Blutflussrichtung ermittelt wird,
FIG 6 eine Bildaufnahme eines Aneurysmas im Bereich der Iliakalarterien,
FIG 7 eine schematische Darstellung einer Kontrastmittelverteilung in einem Bein eines Menschen,
FIG 8 eine Röntgendarstellung eines Gehirns eines Menschen in vertikaler Richtung,
FIG 9 eine schematische Darstellung eines Bilddatengewinnungssystems gemäß einem Ausführungsbeispiel der Erfindung.

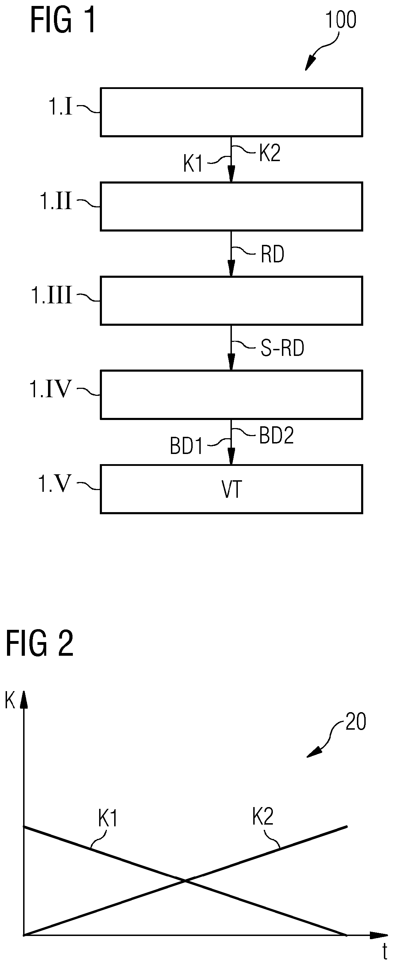

In FIG 1 ist ein Flussdiagramm 100 gezeigt, welches ein Bildgebungsverfahren gemäß einem Ausführungsbeispiel der Erfindung veranschaulicht. Bei dem Schritt 1.I wird zunächst eine Konzentration-Zeit-Kurve für eine Injektion eines Kontrastmittelgemischs mit mindestens zwei spektral unterscheidbaren Kontrastmitteln mit einer vorbestimmten, zeitlich variablen Mischkonzentration festgelegt. D.h., es werden zwei Konzentration-Zeit-Kurven für zwei unterschiedliche Kontrastmittel festgelegt, die bezüglich ihrer spektralen Kontrastwirkung voneinander abweichen. Beispielsweise wird festgelegt, dass die Injektionsmenge eines ersten Kontrastmittels K1 bei dem Start der Injektion ein Maximum aufweist und mit der Zeit abnimmt und die Injektionsmenge K2 eines zweiten Kontrastmittels K2 bei dem Start der Injektion ein Minimum aufweist und mit der Zeit zunimmt. Eine solche Konzentration-Zeit-Kurve ist in FIG 2 gezeigt.

Bei dem Schritt 1.II werden nach einer dem festgelegten Vorgehen entsprechenden Kontrastmittelgabe Röntgenrohdaten RD von einem von dem Kontrastmittelgemisch durchfluteten Bereich eines Untersuchungsobjekts, beispielsweise ein Patient, aufgenommen.

Weiterhin erfolgt bei dem Schritt 1.III eine Spektralzerlegung auf Basis der Röntgenrohdaten RD bezüglich der unterschiedlichen Kontrastmittel K1, K2, wobei spektralzerlegte Rohdaten S-RD gewonnen werden.

Anschließend werden bei dem Schritt 1.IV zwei Bilddatensätze BD1, BD2 auf Basis der spektralzerlegten Rohdaten S-RD gewonnen. Die Zerlegung der Rohdaten hinsichtlich unterschiedlicher Spektren kann zum Beispiel direkt durch eine spektral getrennte Akquisition der Rohdaten mit unterschiedlichen Spektren zugeordneten voneinander separierten Sensoren realisiert werden. Die Rohdaten können aber auch zunächst gemeinsam mit denselben Sensoren erfasst werden und anschließend spektral zerlegt werden.

Schließlich erfolgt bei dem Schritt 1.V ein Ermitteln von funktionellen Daten VT auf Basis der beiden Bilddatensätze BD1, BD2.

In FIG 2 ist ein Schaubild 20 gezeigt, welches den zeitlichen Verlauf der Konzentration K zweier in einem Patienten zu injizierender Kontrastmittel K1, K2 zeigt. Dabei erfolgt eine gleichzeitige Gabe der beiden Kontrastmittel K1, K2 mit zeitlich veränderlicher Konzentration. Bei dem Start einer Injektion ist die Konzentration eines ersten Kontrastmittels K1 maximal und die Konzentration eines zweiten Kontrastmittels K2 minimal. Im weiteren Zeitverlauf sinkt die Konzentration des ersten Kontrastmittels K1 immer weiter ab, dafür nimmt die Konzentration des zweiten Kontrastmittels K2 zu. Die Gesamtkonzentration der beiden Kontrastmittel K1, K2 bleibt bei dem in FIG 2 gezeigten Ausführungsbeispiel stets gleich, so dass eine Bildgebung mit einer homogenen Gesamtverteilung der beiden Kontrastmittel erfolgen kann.

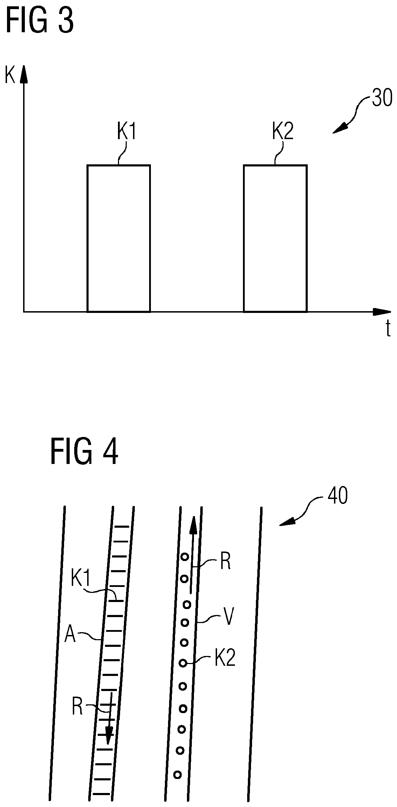

In FIG 3 ist ein Schaubild 30 gezeigt, welches den zeitlichen Verlauf der Konzentration K zweier für eine Bildgebung anzuwendender Kontrastmittel K1, K2 gemäß einem zweiten Ausführungsbeispiel der Erfindung veranschaulicht. Bei dem zweiten Ausführungsbeispiel erfolgt in einem ersten Zeitraum eine Kontrastmittelgabe eines ersten Kontrastmittels K1 mit einer konstanten Konzentration. Nach dem Ende der Gabe des ersten Kontrastmittels K1 wird nach einer Pause ein zweites Kontrastmittel K2 ebenfalls mit einer konstanten Konzentration gegeben. Es erfolgt also eine zeitlich versetzte Gabe der beiden Kontrastmittel.

In FIG 4 ist eine schematische Darstellung eines Körperbereichs 40, in diesem Fall ein Bein, mit Arterien A und Venen V dargestellt. Die Blutflussrichtung R in den Arterien A und den Venen V ist entgegengesetzt. Erfolgt nun eine zeitversetzte Kontrastmittelgabe, wie sie in FIG 3 veranschaulicht ist und wird der Aufnahmezeitpunkt eines Röntgenbilds von dem Körperbereich 40 so gewählt, dass sich das erste bzw. früher verabreichte Kontrastmittel K1 bereits im venösen Kreislauf befindet, während sich das zweite später verabreichte Kontrastmittel K2 im arteriellen Kreislauf befindet, so können Arterien A und Venen V durch eine spektrale Röntgenbildaufnahme voneinander getrennt dargestellt werden. Dazu können zum Beispiel zwei getrennte Bilddatensätze erzeugt werden und durch eine getrennte Darstellung der beiden Bilddatensätze können Arterien A und Venen V getrennt dargestellt werden. Alternativ können die beiden Bilddatensätze auch zu einem Mischbild aus allen Spektren kombiniert werden, wobei Arterien und Venen unterschiedlich eingefärbt werden. Auf diese Weise können Arterien A und Venen V in einem gemeinsamen Bild unterschiedlich farbig kodiert dargestellt werden.

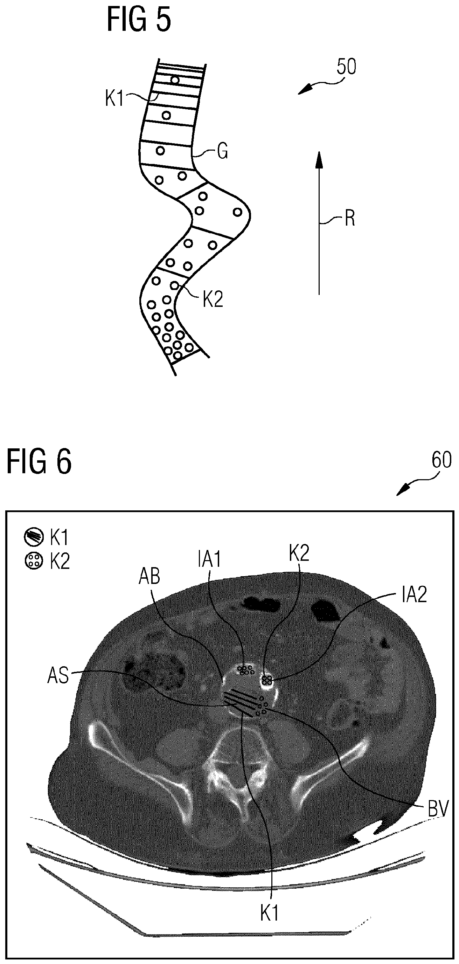

In FIG 5 ist ein Blutgefäß gezeigt, bei dem zwei Kontrastmittel K1, K2 in Abhängigkeit vom Abstand zu dem Injektionsbereich mit unterschiedlichen Kontrastmittelkonzentrationen vorliegen. Die Anwendung zweier Kontrastmittel soll bei diesem Ausführungsbeispiel dazu genutzt werden, eine Blutflussrichtung in dem gezeigten Gefäß zu ermitteln. Die Blutflussrichtung kann zum Beispiel bei einem Vorliegen einer Dissektion nicht eindeutig bekannt sein. Eine Dissektion ist in diesem Zusammenhand eine Aufspaltung der Wandschichten eines Blutgefäßes. Ein erstes Kontrastmittel K1, welches mit Strichen symbolisiert ist, wobei enger benachbarte Striche eine höhere Konzentration des ersten Kontrastmittels K1 darstellen sollen, weist im oberen Bereich des Gefäßes G eine hohe Konzentration auf und im unteren Bereich des Gefäßes G eine niedrige Konzentration auf. Umgekehrt weist ein zweites Kontrastmittel K2, welches durch Kreise symbolisiert ist, wobei enger benachbarte Kreise eine höhere Konzentration des zweiten Kontrastmittels K2 darstellen, eine höhere Konzentration im unteren Bereich des Gefäßes G und eine niedrigere Konzentration im oberen Bereich des Gefäßes G auf. Dabei soll "oben" und "unten" der jeweiligen Position in FIG 5 bei bestimmungsgemäßer Ausrichtung der Zeichnung entsprechen. Der Verlauf der Kontrastmittelkurve der beiden Kontrastmittel soll wie in FIG 2 gezeigt aussehen. Durch die zeitlich konstante Gesamtkonzentration der beiden Kontrastmittel K1, K2 lässt sich eine vollständige homogene Kontrastierung des zu untersuchenden Gefäßes erreichen. Gleichzeitig kann durch eine spektrale Bildgebung das sich zeitlich ändernde Konzentrationsverhältnis der beiden Kontrastmittel K1, K2 ermittelt werden. Dieses Konzentrationsverhältnis kann zum Beispiel farblich kodiert werden.

Weiterhin kann bei einer Bildgebung aus einer erhöhten Konzentration des ersten Kontrastmittels K1 und einer geringen Konzentration des zweiten Kontrastmittels K2 im oberen Bereich und einer niedrigen Konzentration des ersten Kontrastmittels K1 und einer erhöhten Konzentration des zweiten Kontrastmittels K2 im unteren Bereich des Gefäßes eine Flussrichtung R des Bluts in dem Gefäß G von der hohen Konzentration des zweiten Kontrastmittels K2 zu der hohen Konzentration des ersten Kontrastmittels K1 hin ermittelt werden. In dem in FIG 5 gezeigten konkreten Beispiel ist die Blutflussrichtung R von unten nach oben gerichtet.

In FIG 6 ist eine Röntgenaufnahme 60 eines Menschen in Vertikalrichtung gezeigt. In dem Aortenbereich AB, ungefähr im Zentrum des Bildes, ist ein Aortenaneurysma AS gezeigt, welches durch ein erstes Kontrastmittel K1 eingefärbt ist. Zwei Iliakalarterien IA1, IA2, welche jeweils mit einem Stent versorgt sind, sind dagegen durch ein zweites Kontrastmittel K2 eingefärbt. Bei einer gegebenen Kontrastmittelkurve, wie sie in FIG 3 veranschaulicht ist, folgt aus der Kontrastmittelverteilung, dass der Aneurysmasack AS erst später durchblutet wird als die Iliakalarterien IA1, IA2, denn in dem Bereich des Aneurysmasacks AS befindet sich nur das erste Kontrastmittel K1, welches zeitlich früher gegeben wurde als das zweite Kontrastmittel K2. Weiterhin ist rechts neben dem Aneurysmasack AS ein Bereich BV zu erkennen, welcher ebenfalls durch das zweite Kontrastmittel K2 sichtbar gemacht wird. Die Position dieses Bereichs BV rechts neben dem Aneurysma AS deutet darauf hin, dass das Aneurysma AS von rechts aus versorgt wird. Die veranschaulichte Kontrastmittelverteilung lässt also darauf schließen, dass ein Leck, auch als Endoleak bezeichnet, zwischen einem der beiden Stents der Iliakalarterien IA1, IA2 und dem Aneurysmasack AS, und zwar rechts von dem Aneurysma AS liegend, vorliegt. In dem in FIG 6 veranschaulichten Ausführungsbeispiel wird die Darstellung der unterschiedlichen Kontrastmittel K1, K2 dazu genutzt, zu ermitteln, aus welcher Richtung und von welcher Position aus der Aneurysmasack AS mit Blut versorgt wird.

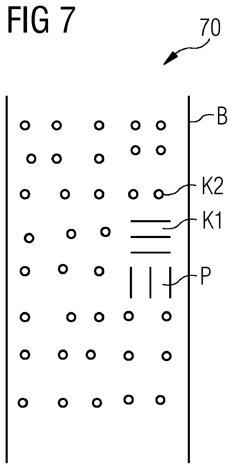

In FIG 7 ist eine schematische Darstellung 70 der Kontrastmittelverteilung in einem Bein B eines Menschen bei einem Bildgebungsverfahren gemäß einem Ausführungsbeispiel der Erfindung veranschaulicht. Die Kontrastmittelgabe wird gemäß der in FIG 3 gezeigten Kontrastmittelzeitkurve durchgeführt. FIG 7 zeigt die Kontrastmittelverteilung zum Zeitpunkt einer spektralen Röntgenbildaufnahme von dem Bein. Die Röntgenbildaufnahme erfolgt erst, nachdem sich das zweite Kontrastmittel K2, welches durch Kreise symbolisiert ist, bereits überall im gut durchbluteten Teil des Beins verteilt hat. Schlechter durchblutete Bereiche des Beins, in denen das Blut aufgrund von Kalkablagerungen oder Engstellen verspätet ankommt, weisen überwiegend das erste Kontrastmittel K1, welches durch horizontale Striche symbolisiert ist, auf. Ein überhaupt nicht mehr durchbluteter Bereich P ist in FIG 7 mit vertikalen parallelen Strichen markiert. Dort befindet sich überhaupt kein Kontrastmittel. Erfolgt die Bildaufnahme ausreichend spät, so sind sämtliche Bereiche, die überhaupt noch durchblutet sind, homogen kontrastiert. Zudem lässt sich aus dem Auftreten des ersten Kontrastmittels K1 auf eine Durchblutungsminderung in dem betreffenden Bereich schließen.

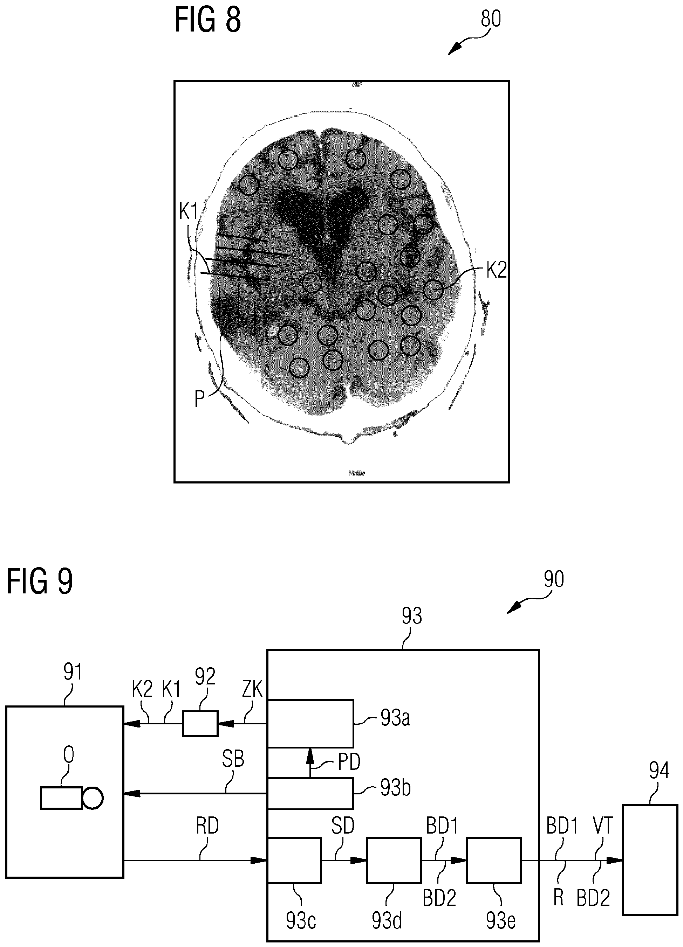

In FIG 8 ist eine Röntgendarstellung 80 eines Schädels eines Menschen in vertikaler Richtung gezeigt. Das in FIG 8 veranschaulichte Verfahren entspricht der in FIG 7 illustrierten Vorgehensweise. Wie in FIG 8 zu erkennen ist, gibt es Bereiche des Gehirns, in denen ein erstes Kontrastmittel K1 vorherrscht, diese sind horizontal gestrichelt gemustert. Weiterhin gibt es Bereiche, in denen ein zweites Kontrastmittel K2 vorherrscht, welches mit Kreisen gekennzeichnet ist. Überdies gibt es einen Bereich P im Gehirn, welcher in FIG 8 durch vertikale Striche schraffiert ist und der weder von dem ersten Kontrastmittel K1 noch von dem zweiten Kontrastmittel K2 durchsetzt ist. Es kann nun aus den unterschiedlichen Konzentrationen der Kontrastmittel K1, K2 gefolgert werden, dass dort, wo überhaupt kein Kontrastmittel angekommen ist, eine Blutversorgung des Gehirns, beispielsweise aufgrund eines Schlaganfalls, nicht mehr möglich ist und die Gehirnsubstanz in diesem Bereich daher nicht mehr zu retten ist. In dem Bereich, in dem jedoch das erste Kontrastmittel K1 vorherrscht, liegt eine lediglich verzögerte Blutversorgung vor. Die Funktion des Gehirns in diesem Bereich kann durch medizinische Maßnahmen möglichweise wiederhergestellt werden. Die Bereiche des Gehirns, in dem das zweite Kontrastmittel K2 vorherrscht, sind von dem Schlaganfall nicht betroffen.

In FIG 9 ist eine schematische Darstellung eines Bilddatengewinnungssystems 90 gemäß einem Ausführungsbeispiel der Erfindung gezeigt. Das Bilddatengewinnungssystem 90 weist ein Röntgensystem 91 auf, mit dem eine Bildaufnahme von einem Patienten O durchgeführt werden kann. Weiterhin umfasst das Bilddatengewinnungssystem 90 eine Steuerungseinrichtung 93. Die Steuerungseinrichtung 93 dient der Ansteuerung des Röntgensystems 91 sowie der Auswertung von von dem Röntgensystem 91 erfassten Rohdaten RD, welche bei der Bildaufnahme erzeugt werden. Teil der Steuerungseinrichtung 93 ist auch eine Injektionsplanungseinheit 93a zum Festlegen einer Konzentration-Zeit-Kurve ZK. Die Konzentration-Zeit-Kurve ZK legt für eine Injektion den zeitlichen Verlauf der Konzentrationen der verabreichten Kontrastmittel fest. Mit der Injektionsplanungseinheit 93a wird eine Injektionseinheit 92 angesteuert, d.h., es wird die ermittelte Konzentrationszeitkurve ZK an die Injektionseinheit 92 übermittelt. Die Steuerungseinrichtung 93 umfasst auch eine Ansteuerungseinheit 93b, mit der das Röntgensystem 91 über Steuerbefehle SB angesteuert wird. Die Ansteuereinheit 93b kann zusätzlich die Injektionsplanungseinheit 93a steuern, um die Festlegung der Konzentration-Zeit-Kurve ZK in Abhängigkeit von Bildgebungsparametern festzulegen. Hierzu können zum Beispiel Protokolldaten PD einer vorzunehmenden Messung von der Ansteuerungseinheit 93b an die Injektionsplanungseinheit 93a übermittelt werden.

Teil der Steuerungseinrichtung 93 ist auch eine Auswertungseinheit 93c zum Durchführen einer Spektralzerlegung auf Basis der Röntgenrohdaten RD bezüglich der unterschiedlichen Kontrastmittel K1, K2. Weiterhin umfasst die Steuerungseinrichtung 93 eine Rekonstruktionseinheit 93d zum Rekonstruieren von zwei Bilddatensätzen BD1, BD2 auf Basis der Spektralzerlegung. Schließlich umfasst die Steuerungseinrichtung 93 auch eine Datenermittlungseinheit 93e zum Ermitteln von funktionellen Daten VT, wie zum Beispiel der Blutflussrichtung R, der Durchblutung oder der Blutflussgeschwindigkeit, auf Basis der mindestens zwei Bilddatensätze BD1, BD2. Die ermittelten funktionellen Daten VT, R können einem Benutzer des Bilddatengewinnungssystems 90 gegebenenfalls in Verbindung mit den erfassten Bilddaten BD1, BD2 über eine Anzeigeeinheit 94 bildlich dargestellt werden.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Bildgebungsverfahren, aufweisend die Schritte:
- Festlegen einer Konzentration-Zeit-Kurve (ZK) für eine Injektion von mindestens zwei spektral unterscheidbaren Kontrastmitteln (K1, K2) mit einer vorbestimmten zeitlich variablen Konzentration,
- Aufnehmen von Röntgenrohdaten (RD) von einem von den Kontrastmitteln (K1, K2) durchfluteten Bereich eines Untersuchungsobjekts (O),
- Durchführen einer Spektralzerlegung auf Basis der Röntgenrohdaten (RD) bezüglich der unterschiedlichen Kontrastmittel (K1, K2),
- Rekonstruieren von mindestens zwei Bilddatensätzen (BD1, BD2) auf Basis der Spektralzerlegung,
- Ermitteln von funktionellen Daten (VT) auf Basis der mindestens zwei Bilddatensätze (BD1, BD2),
wobei durch die Konzentration-Zeit-Kurve (ZK) ein Kontrastmittelgemisch der beiden spektral unterscheidbaren Kontrastmittel (K1, K2) mit einer vorbestimmten zeitlich variablen Mischkonzentration festgelegt wird.

2. Verfahren nach Anspruch 1, wobei durch die Konzentration-Zeit-Kurve (ZK) festgelegt wird, dass die beiden Kontrastmittel (K1, K2) zeitlich versetzt zueinander abgegeben werden.

3. Verfahren nach einem der vorstehenden Ansprüche, wobei die funktionellen Daten (VT) aus einer räumlichen Variation der Konzentration der Kontrastmittel (K1, K2) abgeleitet werden.

4. Verfahren nach einem der vorstehenden Ansprüche, wobei die funktionellen Daten (VT) eine dynamische Messgröße umfassen.

5. Verfahren nach einem der vorstehenden Ansprüche, wobei die funktionellen Daten (VT) eine räumliche Verteilung von Arterien und Venen umfassen.

6. Verfahren nach Anspruch 4, wobei die dynamische Messgröße eine Blutflussgeschwindigkeit umfasst.

7. Verfahren nach einem der vorstehenden Ansprüche, wobei die funktionellen Daten eine Flussrichtung (R) in einem Gefäß (A, V) wiedergeben.

8. Verfahren nach einem der Ansprüche 1 bis 4, wobei die funktionellen Daten (VT) Schädelperfusionsmessdaten umfassen.

9. Verfahren nach Anspruch 4, wobei die dynamische Messgröße einen Bypassfluss oder einen Ruckfluss durch ein Leck in einem Gefäßsystem oder Organ umfasst.

10. Bilddatengewinnungssystem (90), aufweisend:
- eine Injektionsplanungseinheit (93) zum Festlegen einer Konzentration-Zeit-Kurve (ZK) für eine Injektion von mindestens zwei spektral unterscheidbaren Kontrastmitteln (K1, K2) mit einer vorbestimmten zeitlich variablen Konzentration, wobei durch die Konzentration-Zeit-Kurve (ZK) ein Kontrastmittelgemisch der beiden spektral unterscheidbaren Kontrastmittel (K1, K2) mit einer vorbestimmten zeitlich variablen Mischkonzentration festgelegt wird,
- ein Röntgensystem (91) zum Aufnehmen von Röntgenrohdaten (RD) von einem von den Kontrastmitteln (K1, K2) durchfluteten Bereich eines Untersuchungsobjekts (O),
- eine Auswertungseinheit (93a) zum Durchführen einer Spektralzerlegung auf Basis der Röntgenrohdaten (RD) bezüglich der unterschiedlichen Kontrastmittel (K1, K2),
- eine Rekonstruktionseinheit (93b) zum Rekonstruieren von mindestens zwei Bilddatensätzen (BD1, BD2) auf Basis der Spektralzerlegung,
- eine Datenermittlungseinheit (93c) zum Ermitteln von funktionellen Daten (VT) auf Basis der mindestens zwei Bilddatensätze (BD1, BD2).

11. System nach Anspruch 10, wobei das Röntgensystem (91) ein CT-System umfasst.

12. Computerprogrammprodukt mit einem Computerprogramm, welches direkt in eine Speichereinrichtung eines Bilddatengewinnungssystems (90) ladbar ist, mit Programmabschnitten, um alle Schritte eines Verfahrens nach einem der Ansprüche 1 bis 9 auszuführen, wenn das Computerprogramm in dem Bilddatengewinnungssystem (90) ausgeführt wird.

13. Computerlesbares Medium, auf welchem von einer Rechnereinheit einlesbare und ausführbare Programmabschnitte gespeichert sind, um alle Schritte eines Verfahrens nach einem der Ansprüche 1 bis 9 auszuführen, wenn die Programmabschnitte von der Rechnereinheit ausgeführt werden.

## Claims

1. Imaging method, having the steps:
- defining a concentration time curve (ZK) for an injection of at least two spectrally differentiable contrast agents (K1, K2) with a predetermined temporally variable concentration,
- recording x-ray raw data (RD) of a region of an examination object (O) through which the contrast agents (K1, K2) flow,
- carrying out a spectral decomposition on the basis of the x-ray raw data (RD) with respect to the different contrast agents (K1, K2),
- reconstructing at least two image data sets (BD1, BD2) on the basis of the spectral decomposition,
- determining functional data (VT) on the basis of the at least two image data sets (BD1, BD2),
wherein by means of the concentration time curve (ZK) a contrast agent mixture of the two spectrally differentiable contrast agents (K1, K2) is defined with a predetermined temporally variable mixed concentration.

2. Method according to claim 1, wherein by means of the concentration time curve (ZK) it is defined that the two contrast agents (K1, K2) are output in a temporally offset manner with respect to one another.

3. Method according to one of the preceding claims, wherein the functional data (VT) is derived from a spatial variation of the concentration of the contrast agents (K1, K2).

4. Method according to one of the preceding claims, wherein the functional data (VT) comprises a dynamic measured value.

5. Method according to one of the preceding claims, wherein the functional data (VT) comprises a spatial distribution of arteries and veins.

6. Method according to claim 4, wherein the dynamic measured value comprises a blood flow speed.

7. Method according to one of the preceding claims, wherein the functional data reproduces a flow direction (R) in a vessel (A, V).

8. Method according to one of claims 1 to 4, wherein the functional data (VT) comprises cranial perfusion measurement data.

9. Method according to claim 4, wherein the dynamic measured value comprises a bypass flow or a return flow through a leak in a vascular system or organ.

10. Image data extraction system (90), having:
- an injection planning unit (93) for defining a concentration time curve (ZK) for an injection of at least two spectrally differentiable contrast agents (K1, K2) with a predetermined temporally variable concentration, wherein by means of the concentration time curve (ZK) a contrast agent mixture of the two spectrally differentiable contrast agents (K1, K2) is defined with a predetermined temporally variable mixed concentration,
- an x-ray system (91) for recording x-ray raw data (RD) of a region of an examination object (O) through which the contrast agents (K1, K2) flow,
- an evaluation unit (93a) for carrying out a spectral decomposition on the basis of the x-ray raw data (RD) with respect to the different contrast agents (K1, K2),
- a reconstruction unit (93b) for reconstructing at least two image data sets (BD1, BD2) on the basis of the spectral decomposition,
- a data determination unit (93c) for determining functional data (VT) on the basis of the at least two image data sets (BD1, BD2).

11. System according to claim 10, wherein the x-ray system (91) comprises a CT system.

12. Computer program product having a computer program, which can be loaded directly into a storage facility of an image data extraction system (90), having program sections, in order to execute all steps of a method as claimed in one of claims 1 to 9, if the computer program is executed in the image data extraction system (90).

13. Computer readable medium, on which program sections which can be read in and executed by a computer unit are stored in order to execute all steps of a method as claimed in one of claims 1 to 9 if the program sections are executed by the computer unit.

## Revendications

1. Procédé d'imagerie, comportant les stades :
- on fixe une courbe (ZK) concentration-temps pour une injection d'au moins deux agents (K1, K2) de contraste pouvant être distingués spectralement en ayant une concentration variable dans le temps définie à l'avance,
- on enregistre des données (RD) brutes de rayons X par une partie d'un objet (O) à étudier irriguée par les agents (K1, K2) de contraste,
- on effectue une décomposition spectrale sur la base des données (RD) brutes de rayons X rapportée aux agents (K1, K2) de contraste différents,
- on reconstruit au moins deux ensembles (BD1, BD2) de données d'image sur la base de la décomposition spectrale,
- on détermine des données (VT) fonctionnelles sur la base des au moins deux ensembles (BD1, BD2) de données d'image,
dans lequel par la courbe (ZK) concentration-temps, on fixe un mélange des deux agents (K1, K2) de contraste pouvant être distingués spectralement en ayant une concentration de mélange variable dans le temps définie à l'avance.

2. Procédé suivant la revendication 1, dans lequel, par la courbe (ZK) concentration-temps, on fixe que les deux agents (K1, K2) de contraste sont administrés d'une manière décalée dans le temps l'un par rapport à l'autre.

3. Procédé suivant l'une des revendications précédentes, dans lequel on déduit les données (VT) fonctionnelles d'une variation dans l'espace de la concentration des agents (K1, K2) de contraste.

4. Procédé suivant l'une des revendications précédentes, dans lequel les données (VT) fonctionnelles comprennent une grandeur de mesure dynamique.

5. Procédé suivant l'une des revendications précédentes, dans lequel des données (VT) fonctionnelles comprennent une répartition dans l'espace d'artères et de veines.

6. Procédé suivant la revendication 4, dans lequel la grandeur de mesure dynamique comprend une vitesse du flux sanguin.

7. Procédé suivant l'une des revendications précédentes, dans lequel les données fonctionnelles reproduisent un sens (R) du flux dans un vaisseau (A, V).

8. Procédé suivant l'une des revendications 1 à 4, dans lequel les données (VT) fonctionnelles comprennent des données de perfusion céphalométriques.

9. Procédé suivant la revendication 4, dans lequel la grandeur de mesure dynamique comprend un flux de dérivation ou un flux de retour par une fuite dans un système de vaisseaux ou un organe.

10. Système (90) d'obtention de données d'image, comportant :
- une unité (93) de planification d'injection pour la fixation d'une courbe (ZK) concentration-temps d'une injection d'au moins deux agents (K1, K2) de contraste pouvant être distingués spectralement en ayant une concentration variant dans le temps définie à l'avance, dans lequel, par la courbe (ZK) concentration-temps, on fixe un mélange des deux agents (K1, K2) de contraste pouvant être distingués spectralement en ayant une concentration du mélange variant dans le temps définie à l'avance,
- un système (91) à rayons X pour l'enregistrement de données (RD) brutes de rayons X d'une partie d'un objet (O) à étudier irriguée par les agents (K1, K2) de contraste,
- une unité (93a) d'analyse pour effectuer une décomposition spectrale sur la base des données (RD) brutes de rayons X rapportée aux agents (K1, K2) de contraste différents,
- une unité (93b) de reconstruction pour la reconstruction d'au moins deux ensembles (BD1, BD2) de données d'image sur la base de la décomposition spectrale,
- une unité (93c) de détermination de données pour la détermination de données (VT) fonctionnelles sur la base des au moins deux ensembles (BD1, BD2) de données d'image.

11. Système suivant la revendication 10, dans lequel le système (91) à rayons X comprend un système CT.

12. Produit de programme d'ordinateur comprenant un programme d'ordinateur, qui peut être chargé directement dans un dispositif de mémoire d'un système (90) d'obtention de données d'image, comprenant des parties de programme pour effectuer tous les stades d'un procédé suivant l'une des revendications 1 à 9, lorsque le programme d'ordinateur est réalisé dans le système (90) d'obtention de données d'image.

13. Support, déchiffrable par ordinateur, sur lequel sont mises en mémoire des parties de programme pouvant être lues et réalisées par une unité informatique afin d'effectuer tous les stades d'un procédé suivant l'une des revendications 1 à 9, lorsque les parties du programme sont réalisées par l'unité informatique.
